Europäisches Patentamt

European Patent Office

Office européen des brevets

(11) Veröffentlichungsnummer: **0 074 002**
**B1**

(12) # EUROPÄISCHE PATENTSCHRIFT

(45) Veröffentlichungstag der Patentschrift:
12.03.86

(51) Int. Cl.⁴: **C 07 C 127/15**

(21) Anmeldenummer: **82107685.8**

(22) Anmeldetag: **23.08.82**

(54) **Verfahren zur Herstellung von Monocyclohexylharnstoff.**

(30) Priorität: **04.09.81 DE 3135111**

(43) Veröffentlichungstag der Anmeldung:
**16.03.83 Patentblatt 83/11**

(45) Bekanntmachung des Hinweises auf die Patenterteilung:
**12.03.86 Patentblatt 86/11**

(84) Benannte Vertragsstaaten:
**BE CH DE FR GB IT LI NL**

(56) Entgegenhaltungen:
**EP - A - 0 025 548**
**DE - B - 1 468 774**

**FERRI "Reaktionen der organischen Synthese", 1978**
**GEORG THIEME VERLAG, Stuttgart Seite 658 (8)**
**ULLMANN'S "Encycklopädie der technischen Chemie",**
**4. Auflage, Band 12, 1976 VERLAG CHEMIE,**
**Weinheim-New York Seite 508, rechte Spalte, Zeilen**
**17-21**

(73) Patentinhaber: **BAYER AG, Konzernverwaltung RP**
**Patentabteilung, D-5090 Leverkusen 1 Bayerwerk (DE)**

(72) Erfinder: **Fauss, Rudolf, Dr., Gerstenkamp 10,**
**D-5000 Köln 80 (DE)**
Erfinder: **Findeisen, Kurt, Dr., In der Follmuehle 10,**
**D-5068 Odenthal 2 (DE)**
Erfinder: **Döbereiner, Uwe, Dr., Scheurener Strasse 87,**
**D-5068 Odenthal (DE)**

Beschreibung

Die Erfindung betrifft ein Verfahren zur Herstellung von Monocyclohexylharnstoff.

Aus der US 2 253 528 und der US 2 257 717 ist bekannt, Monocyclohexylharnstoff durch Umsetzung von Cyclohexylamin mit einem Überschuss an Harnstoff und bei einem Überdruck in einem Autoklaven in einem Temperaturbereich von 100 °C bis zum Zersetzungspunkt des Monocyclohexylharnstoffs herzustellen. Hierbei entstehen erhebliche Anteile an Dicyclohexylharnstoff, der in aufwendiger Weise abgetrennt werden muss.

Aus der DE-PS 855 551 ist bekannt, Monomethylharnstoff durch Umsetzung von Harnstoff mit einer molekularen Menge an wässrigem Methylamin unter Druck im Temperaturbereich von 110 bis 120 °C herzustellen. Auch bei diesem Verfahren entsteht der schlecht abtrennbare Dimethyl-harnstoff.

Aus Ullmanns Enzyklopädie der technischen Chemie Band 8, S. 389 (1957) ist bekannt, dass Monoalkylharnstoffe durch Umsetzung von Harnstoff mit primären aliphatischen Aminen in wässriger Lösung in der Wärme hergestellt werden können. Diese Umsetzung lässt sich nur bei der Herstellung von Monomethyl- und Monoethylharnstoff durchführen.

Es wurde ein Verfahren zur Herstellung von Monocyclohexylharnstoff durch Umsetzung von Harnstoff mit Cyclohexylamin bei erhöhter Temperatur gefunden, das dadurch gekennzeichnet ist, dass man 100 Gew.-Teile des Cyclohexylamins mit 70 bis 300 Gew.-Teilen Harnstoff in Gegenwart von 100 bis 500 Gew.-Teilen Wasser im Siedebereich des Wassers umsetzt.

Als Harnstoff für das erfindungsgemässe Verfahren kann auch sogenannter technischer Harnstoff verwendet werden, der Biuret enthält. Durch das Biuret wird das Ergebnis des erfindungsgemässen Verfahrens nicht verändert.

Cyclohexylamin wird im allgemeinen durch Hydrieren von Anilin hergestellt.

Erfindungsgemäss setzt man 100 Gew.-Teile des Cyclohexylamins mit 70 bis 300 Gew.-Teilen Harnstoff in Gegenwart von 100 bis 500 Gew.-Teilen Wasser um. Bevorzugt wird die Umsetzung von 100 Gew.-Teilen Cyclohexylamin mit 75 bis 120 Gew.-Teilen Harnstoff in Gegenwart von 120 bis 200 Gew.-Teilen Wasser.

In einer bevorzugten Ausführungsform des erfindungsgemässen Verfahrens legt man zu Beginn der Umsetzung 40 bis 60 Gew.-Teile des Wassers vor und gibt dann den Harnstoff und das Cyclohexylamin hinzu. Den Rest des Wassers ergänzt man im allgemeinen bei dem ersten Auftreten eines Niederschlags von Monocyclohexylharnstoff in dem Reaktionsgemisch.

Das erfindungsgemässe Verfahren führt man im allgemeinen unter Normaldruck (etwa 1 bar) durch. Erfindungsgemäss erfolgt die Umsetzung im Siedebereich des Wassers. Bevorzugt wird das erfindungsgemässe Verfahren bei Normaldruck im Temperaturbereich von 90 bis 110 °C, insbesondere bevorzugt von 95 bis 105 °C, durchgeführt.

Im allgemeinen führt man das erfindungsgemässe Verfahren wie folgt durch:

Cyclohexylamin und Harnstoff werden in Wasser gelöst und unter Rückfluss erhitzt, wobei sich nach einiger Zeit der in Wasser wenig lösliche Monocyclohexylharnstoff abscheidet. Die Reaktion ist beendet, wenn kein Ammoniak mehr entsteht.

Nach beendigter Umsetzung wird das Reaktionsgemisch auf Raumtemperatur abgekühlt und der Monocyclohexylharnstoff abgesaugt, mit wenig Wasser gewaschen und getrocknet.

Nach dem erfindungsgemässen Verfahren erhält man den Monocyclohexylharnstoff in sehr hohen Ausbeuten und mit hoher Reinheit. Der Dicyclohexylharnstoff entsteht praktisch nicht als Nebenprodukt.

Das erfindungsgemässe Verfahren lässt sich sowohl kontinuierlich als auch diskontinuierlich durchführen. Die in der Mutterlauge verbliebenen Restmengen an Cyclohexylamin und der Überschuss an Harnstoff können in weiteren Umsetzungen eingesetzt werden.

Es ist selbstverständlich auch möglich, das Amin oder das Wasser während der Umsetzung teilweise zu dosieren. Ebenso kann die Entfernung des entstehenden Ammoniaks durch Ausblasen mit einem Inertgas (z.B. Stickstoff oder Wasserdampf) begünstigt werden.

Auch ist es möglich, die Aufarbeitung des Reaktionsgemisches so zu ändern, dass nach teilweiser Umsetzung ein gewisser Teil des Ansatzes ausgeschleust wird, um den bereits gebildeten Monocyclohexylharnstoff abzutrennen. Auch hier kann dann die Mutterlauge für eine weitere Umsetzung zurückgeführt werden.

Es ist überraschend, dass man nach dem erfindungsgemässen Verfahren selektiv Monocyclohexylharnstoff erhält. Da der Monocyclohexylharnstoff bei Reaktionstemperatur in nicht unerheblichem Mass wasserlöslich ist, wäre eine Weiterreaktion mit Cyclohexylamin zu sehr schwerlöslichen Dicyclohexylharnstoff zu erwarten gewesen. Dieser hätte demnach aus der Reaktionslösung ausfallen und entsprechend dem Massenwirkungsgesetz das Gleichgewicht weiter zu Ungunsten des Monocyclohexylharnstoffes verschieben müssen. Dies geschieht überraschenderweise jedoch nicht.

Monocyclohexylharnstoff kann bei der Herstellung von Harzen und Pflanzenschutzmitteln, in der Pharma- und Farbstoffchemie, als Weichmacher und als Stabilisator verwendet werden (Ullmanns Enzyklopädie der technischen Chemie, 4. Auflage, Band 12 (1976), S. 508).

Beispiel 1

495 g (5 Mol) Cyclohexylamin und 450 g (7,5 Mol) Harnstoff werden in 250 ml Wasser unter Ammoniakabspaltung am Rückfluss erhitzt.

Nach ca. 2 Stunden beginnt N-Cyclohexylharnstoff auszufallen. Im Laufe von weiteren 4 Stunden wird langsam 700 ml Wasser zugegeben.

Es wird noch unter leichtem Rückfluss nachgerührt, bis die Ammoniakentwicklung fast völlig beendet ist.

Nach dem Abkühlen wird abgesaugt und mit Wasser nachgewaschen. Nach dem Trocknen fallen 681 g (= 96% der Theorie, bezogen auf Cyclohexylamin) N-Cyclohexylharnstoff (Fp. 194–195 °C) an. Nachdem DC enthält die Substanz weniger als 2% Dicyclohexylharnstoff.

Beispiel 2

346,5 g (3,5 Mol) Cyclohexylamin und 273 g (4,55 Mol) Harnstoff werden in 175 ml Wasser am Rückfluss erhitzt. Nachdem etwas N-Cyclohexylharnstoff ausgefallen ist, werden langsam 350 ml Wasser zugetropft und weiter am Rückfluss gehalten, bis die Ammoniakabspaltung beendet ist. Nach dem Aufarbeiten wie in Beispiel 1 werden 472,6 g (95,1% der Theorie) N-Cyclohexylharnstoff erhalten.

Beispiel 3

495 g (5 Mol) Cyclohexylamin und 390 g (6,5 Mol) Harnstoff werden in 750 ml Wasser am Rückfluss erhitzt. Nach beendeter Ammoniakabspaltung wird abgekühlt und abgesaugt. Nach Nachwaschen und Trocknen werden 638 g N-Cyclohexylharnstoff (90% der Theorie) erhalten.

Beispiel 4

730 l Mutterlauge aus Beispiel 3, 50 ml Wasser, 495 g (5 Mol) Cyclohexylamin und 312 g (5,2 Mol) Harnstoff werden wie in Beispiel 3 umgesetzt und aufgearbeitet. Es werden 652 g N-Cyclohexylharnstoff (92% der Theorie) erhalten.

**Patentansprüche**

1. Verfahren zur Herstellung von Monocyclohexylharnstoff durch Umsetzung von Harnstoff mit Cyclohexylamin bei erhöhter Temperatur, dadurch gekennzeichnet, dass man 100 Gew.-Teile des Cyclohexylamins mit 70 bis 300 Gew.-Teilen Harnstoff in Gegenwart von 100 bis 500 Gew.-Teilen Wasser im Siedebereich des Wassers umsetzt.

2. Verfahren nach Anspruch 1, dadurch gekennzeichnet, dass man 100 Gew.-Teile des Cyclohexylamins mit 75 bis 120 Gew.-Teilen Harnstoff in Gegenwart von 120 bis 200 Gew.-Teilen Wasser umsetzt.

3. Verfahren nach den Ansprüchen 1 und 2, dadurch gekennzeichnet, dass man zu Beginn der Umsetzung 40 bis 60 Gew.-Teile des Wassers vorlegt, die Reaktanden zugibt und den Rest des Wassers bei dem ersten Auftreten eines Niederschlags von Monocyclohexylharnstoff in dem Reaktionsgemisch ergänzt.

4. Verfahren nach den Ansprüchen 1 bis 3, dadurch gekennzeichnet, dass man die Umsetzung unter Normaldruck im Temperaturbereich von 90 bis 110 °C durchführt.

**Revendications**

1. Procédé de fabrication de monocyclohexylurée par réaction de l'urée avec de la cyclohexylamine à température élevée, caractérisé en ce qu'on fait réagir 100 parties en poids de cyclohexylamine avec 70 à 300 parties en poids d'urée en présence de 100 à 500 parties en poids d'eau dans l'intervalle d'ébullition de l'eau.

2. Procédé selon la revendication 1, caractérisé en ce qu'on fait réagir 100 parties en poids de cyclohexylamine avec 75 à 120 parties en poids d'urée en présence de 120 à 200 parties en poids d'eau.

3. Procédé selon les revendications 1 et 2, caractérisé en ce qu'au début de la réaction on introduit d'avance 40 à 60 parties en poids de l'eau, on ajoute les réactifs et l'on complète le restant d'eau à la première apparition d'un précipité de monocyclohexylurée dans le mélange de réaction.

4. Procédé selon les revendications 1 à 3, caractérisé en ce qu'on exécute la réaction sous la pression normale dans l'intervalle de température de 90 à 110 °C.

**Claims**

1. Process for the preparation of monocyclohexylurea by reacting urea with cyclohexylamine at an elevated temperature, characterised in that 100 parts by weight of cyclohexylamine are reacted with 70 to 300 parts by weight of urea in the presence of 100 to 500 parts by weight of water in the boiling range of water.

2. Process according to Claim 1, characterised in that 100 parts by weight of cyclohexylamine are reacted with 75 to 120 parts by weight of urea in the presence of 120 to 200 parts by weight of water.

3. Process according to Claims 1 and 2, characterised in that 40 to 60 parts by weight of water are initially introduced at the start of the reaction, the reactants are added and the remainder of the water is added on the first appearance in the reaction mixture of a precipitate of monocyclohexylurea.

4. Process according to Claims 1 to 3, characterised in that the reaction is carried out under normal pressure within a temperature range from 90 to 110 °C.